# EUROPÄISCHE PATENTSCHRIFT

(11) **EP 1 742 575 B1**
(45) Veröffentlichungstag und Bekanntmachung des Hinweises auf die Patenterteilung: **13.10.2010**
(21) Anmeldenummer: 05740702.5
(22) Anmeldetag: 29.04.2005
(51) Int. Cl.: A61B 5/15, B65D 75/34

(54) **TESTMAGAZIN UND VERFAHREN ZU DESSEN VERARBEITUNG**
TEST MAGAZINE AND METHOD FOR USING THEM
CHARGEUR D'ESSAI ET PROCEDE D'UTILISATION CORRESPONDANT

(30) Priorität: 30.04.2004 US 836578
(43) Veröffentlichungstag der Anmeldung: 17.01.2007
(73) Patentinhaber: F.HOFFMANN-LA ROCHE AG, 4070 Basel (CH); Roche Diagnostics GmbH, 68305 Mannheim (DE)
(72) Erfinder: CALASSO, Irio, Giuseppe, CH-6415 Arth (CH); KOPP, Martin, CH-6332 Hagendorn (CH); RANEY, Charles, Camdenton, Missouri 65020 (US); ROE, Steven, N., San Mateo, California 94403 (US)
(74) Vertreter: Pfiz, Thomas
(86) Internationale Anmeldenummer: PCT/EP2005/004646
(87) Internationale Veröffentlichungsnummer: WO 2005/104948

(56) Entgegenhaltungen:
- EP-A- 1 360 935
- DE-B1- 2 803 345
- US-A1- 2003 199 904

## Beschreibung

Die Erfindung betrifft ein Testmagazin mit zwei sandwichartig miteinander verbundenen, aufwickelbaren Folienbändern, zwischen denen Aufnahmezellen für Testelemente freigehalten sind, und einer Vielzahl von Testelementen, welche jeweils eine Stecheinheit zum Einstechen in Körpergewebe und eine Testeinheit zur Beaufschlagung mit Körperflüssigkeit umfassen. Die Erfindung betrifft weiter ein Verfahren zur Verarbeitung eines solchen Magazins.

Solche Testsysteme sollen vor allem Diabetikern zur täglich mehrfach durchgeführten Blutzucker-Selbstkontrolle dienen. Neuere Konzepte sehen eine Mikronadel in Verbindung mit einem Testfeld als Einmalsystem (Disposable) vor, um einen Hauteinstich zu erzeugen, daraus eine kleine Menge Blut unter Ausnutzung von Kapillarkräften zu entnehmen und diese Blutprobe zu analysieren. Mit einem solchen integrierten System sollen in einem weitgehend automatischen Messablauf auch von Laien die erforderlichen Schritte einfach und schnell vorgenommen werden können. Ein wichtiger Aspekt ist die Miniaturisierung auch im Hinblick auf eine hohe Integration von Disposables auf engem Raum in einem Handgerät.

In diesem Zusammenhang wurden in der EP-A-1360935 bereits Blisterpackungen für packungsfest integrierte, ein Sensorteil und ein daran abstehendes Stechorgan umfassende Testelemente vorgeschlagen, welche jedoch aufgrund von starren Rahmen mit wannenförmigen Ausformungen Nachteile sowohl bei der Herstellung als auch im Einsatz mit sich bringen.

Ausgehend hiervon liegt der Erfindung die Aufgabe zugrunde, die im Stand der Technik aufgetretenen Nachteile zu vermeiden und Verbesserungen auch dahingehend zu schaffen, dass eine hohe Integrationsdichte und hygienische Handhabung möglich ist.

Zur Lösung dieser Aufgabe werden die in den unabhängigen Patentansprüchen angegebenen Merkmalskombinationen vorgeschlagen. Vorteilhafte Ausgestaltungen und Weiterbildungen der Erfindung ergeben sich aus den abhängigen Ansprüchen.

Die Erfindung geht von dem Gedanken aus, in zweidimensionalen zellenförmigen Bereichen einer Folienpackung eine hohe Integrationsdichte und Handhabungssicherheit für die Testelemente zu gewährleisten. Dementsprechend liegt die Erfindung darin, dass die Stecheinheiten und Testeinheiten in gesonderten Aufnahmezellen voneinander getrennt angeordnet sind. Die Zellen lassen sich einfach durch den Zwischenbereich zwischen den nicht weiter ausgeformten Folienbändern realisieren. Durch die Trennung werden auch Vorteile dahingehend erreicht, dass die Stechelemente ohne Schädigung der Testchemie unabhängig vorbehandelt, insbesondere sterilisiert und hydrophilisiert werden können, und dass beim Stechvorgang keine Gefahr besteht, dass

Testchemikalien in den Körper gelangen. Zudem zeichnet sich diese Lösung durch die Möglichkeit einer einfachen Herstellung insbesondere durch Rolle-zu Rolle-Fertigung aus, wobei ein besonders flacher, wickelfähiger Aufbau möglich ist.

Vorteilhafterweise sind die Stecheinheiten und Testeinheiten in Bandrichtung alternierend oder quer dazu paarweise benachbart in zugeordneten Aufnahmezellen angeordnet.

Eine besonders bevorzugte Ausführung sieht vor, dass die Stecheinheiten in den zugeordneten Aufnahmezellen durch Bestrahlung vorzugsweise über eine Maske zur Abschirmung der Testeinheiten sterilisiert sind.

Eine Fertigungsvereinfachung wird auch dadurch erreicht, dass die Folienbänder über ihre Länge mit einem Testband verbunden sind, und dass das Testband durch Durchbrüche der Folienbänder unter Bildung der Testeinheiten zellenweise freigestellt ist.

Im Hinblick auf eine lange Haltbarkeit ist es auch von Vorteil, wenn in den Aufnahmezellen für die Testeinheiten ein Trockenmittel eingelagert ist.

Ein weiterer Erfindungsaspekt besteht darin, dass zumindest die Stecheinheiten durch Transportmittel aus ihrer jeweiligen Aufnahmezelle entnehmbar und in eine von den Folienbändern vollständig getrennte Arbeitsposition bewegbar sind. Auf diese Weise wird ein vollautomatischer Verfahrensablauf ohne Behinderung durch die Magazinierung möglich, wobei auch die hygienische Entsorgung verbrauchter Einheiten einfach gelöst werden kann.

Für ein einfaches sukzessives Freistellen einzelner Einheiten ist es vorteilhaft, wenn die Transportmittel eine Bandzugvorrichtung zum Auseinanderziehen der Folienbänder in verschiedene Richtungen umfassen. Dies lässt sich dadurch realisieren, dass die Bandzugvorrichtung zwei an einer Spendestelle im seitlichen Abstand voneinander angeordnete, in entgegengesetzte Richtungen drehbare oder feststehende Umlenkzylinder und den Umlenkzylindern nachgeordnete Aufwickelspulen für die Folienbänder aufweist.

Um nach dem Sammelvorgang zu analysierende Körperflüssigkeit einfach weiterverarbeiten zu können, ist es von Vorteil, wenn die Transportmittel zum Rückführen einer Stecheinheit in eine Wirkverbindung mit einer auf den Folienbändern befindlichen Testeinheit und/oder Entsorgungsstelle ausgebildet sind.

Eine weitere Verbesserung wird dadurch erreicht, dass die Transportmittel eine Handhabungsvorrichtung zum Aufgreifen und Positionieren einer durch Auseinanderziehen der Folienbänder an einer Spendestelle freigestellten Stecheinheit umfassen. Hierbei ist es günstig, wenn zumindest eines der Folienbänder Positionierlöcher insbesondere im Bereich der Aufnahmezellen der Testeinheiten für den Eingriff der Handhabungsvorrichtung aufweist.

Die Einzelentnahme und Handhabung eignet sich auch für integrierte Testelemente, bei denen die Stecheinheiten mit den Testeinheiten als körperlich verbunden sind.

Eine besonders bevorzugte Ausgestaltung der Erfindung sieht vor, dass zumindest eines der Folienbänder mit einer Rücknahmestruktur zur vorzugsweise klebenden oder klemmenden Fixierung verbrauchter Stechelemente versehen ist. Auf diese Weise ist eine besonders einfache Entsorgung von kontaminierten Einheiten möglich.

Zur Erhöhung der Integrationsdichte ist es vorteilhaft, wenn die Stecheinheiten als Flachmaterialteile flach zwischen den Folienbändern angeordnet sind. Hierbei sollten die Folienbänder eben ausgebildet sein und frei von Blistern bzw. räumlichen Ausformungen flächig gegen die Stecheinheiten anliegen.

Eine weitere vorteilhafte Ausführung sieht vor, dass die an einer Spendestelle freigestellten Stecheinheiten an einer davon entfernten Rückgabestelle auf eines der Folienbänder zurückdispensiert werden. Damit werden Einschränkungen im Einzelhandling der Stecheinheiten und gegebenenfalls damit verbundener Testelemente weiter vermieden.

Vorteilhafterweise besitzen die Stecheinheiten eine vorzugsweise durch einen halboffenen Kanal gebildete Kapillarstruktur zum Sammeln von Körperflüssigkei. Eine weitere Verbesserung sieht vor, dass die Stecheinheiten zur Weitergabe von aufgenommener Körperflüssigkeit auf die Testeinheiten ausgebildet sind.

Für die Herstellung der Zellenstruktur ist es günstig, wenn die Aufnahmezellen durch linienförmige Folienverbindungen, vorzugsweise Schweiß- oder Klebenähte zwischen den Folienbändern begrenzt sind. Eine weitere Verbesserung wird dadurch erreicht, dass die Aufnahmezellen durch die Folienverbindungen gegeneinander und gegen die Umgebung stoffdicht abgedichtet sind.

Um das Aufreißen der Folienpackung zu erleichtern, ist es vorteilhaft, wenn die linienförmigen Folienverbindungen schräg zur Bandlängsrichtung der Folienbänder verlaufen.

Vorteilhafterweise sind die Stecheinheiten durch formschlüssig umrandende oder in Ausnehmungen eingreifende Folienverbindungen lagefest in ihrer zugeordneten Aufnahmezelle fixiert.

Günstig ist es auch, wenn mindestens eines der Folienbänder ein transparentes Messfenster für eine optische Vermessung der Testeinheiten aufweist oder bildet, so dass ein berührungsloses Abtasten durch das Band hindurch möglich ist.

Eine hohe Packungsdichte lässt sich auch dadurch erreichen, dass die Folienbänder mit den darin befindlichen Testelementen in einer Zickzack-Faltung als Faltpaket bereitgehalten sind.

Vorteilhafterweise sind die Folienbänder mit den darin befindlichen Testelementen in einer Kassette aufgenommen. Die Testeinheiten können speziell als mit Reagenzien beschichtete Nachweisfelder zum Nachweis eines Analyten in der Körperflüssigkeit, insbesondere Glucose ausgebildet sein.

In verfahrensmäßiger Hinsicht wird die eingangs genannte Aufgabe dadurch gelöst, dass die Stecheinheiten gegebenenfalls in Verbindung integrierten Testeinheiten durch Auseinanderziehen der Folienbänder einzeln freigestellt, sodann in eine von der zugehörigen Aufnahmezelle entfernte Arbeitsposition bewegt und anschließend wieder auf einem der Folienbänder remagaziniert werden.

Im Folgenden wird die Erfindung anhand der in der Zeichnung schematisch dargestellten Ausführungsbeispiele näher erläutert. Es zeigen:
Fig. 1 ein Folienmagazin für Blutglucosetests in einer ausschnittsweisen Draufsicht;
Fig. 2 ein weiteres Folien- bzw. Testmagazin in einer ausschnittsweisen Draufsicht;
Fig. 3 die einzelnen Bauelemente des Folienmagazins nach Fig. 2 in einer Explosionsdarstellung;
Fig. 4 und 5 weitere Ausführungsformen von Folienmagazinen in ausschnittsweiser Draufsicht;
Fig. 6 eine Ausführungsform mit einem Faltenbalg als Vorrat für Testelemente in schaubildlicher Darstellung;
Fig. 7 ein Testmagazin in einem Magazingehäuse in aufgebrochener perspektivischer Darstellung; und
Fig. 8 ein Handgerät zur Verarbeitung eines Testmagazins nach Fig. 7.

Die in der Zeichnung dargestellten Testmagazine umfassen als Folienverpackung 10 zwei sandwichartig miteinander verbundene Folienbänder 12, 14, zwischen denen Aufnahmebereiche bzw. -zellen 16 freigehalten sind, in welchen Testelemente 18, 20 zur sukzessiven Verarbeitung bereitgestellt sind.

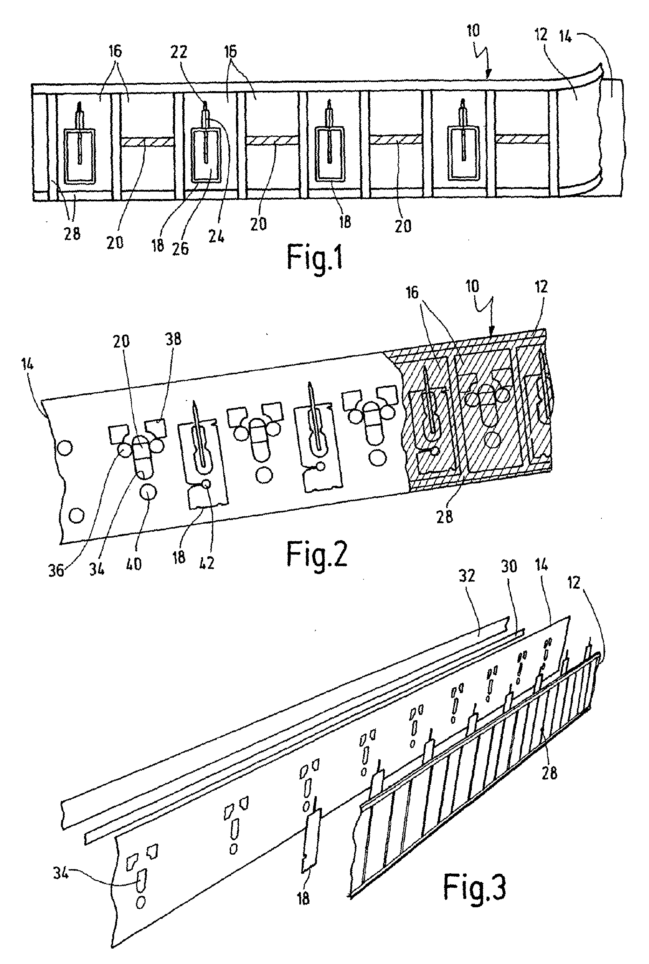

Wie aus Fig. 1 ersichtlich, sind als Testelemente einander paarweise zugeordnete Stecheinheiten 18 und Testeinheiten 20 vorgesehen, welche in gesonderten Aufnahmezellen 16 voneinander getrennt angeordnet sind. In den gezeigten Ausführungsbeispielen sind die Stecheinheiten 18 und Testeinheiten 20 in Bandrichtung gesehen alternierend angeordnet. Denkbar sind auch quer zur Bandrichtung seitlich nebeneinander oder schräg versetzt liegende Anordnungen oder integrierte Kombinationen von Testelementen, bei welchen die Stecheinheiten 18 körperlich mit den Testeinheiten 20 verbunden sind.

Die Stecheinheiten 18 sind als Flachformteile aus einem dünnen Edelstahlblech gebildet und weisen in Form einer Mikronadel ein distales Stechorgan 22 zum Einstechen beispielsweise in den Finger eines Probanden zur Blutgewinnung auf. Das Stechorgan 22 ist über einen halboffenen rillenförmigen Kapillarkanal 24 mit einem Haltebereich 26 zum Sammeln und Weitergeben von Blut verbunden. Zu diesem Zweck lässt sich die betreffende Stecheinheit 18 nach dem Sammelvorgang mit einer zugeordneten Testeinheit 20 so in Verbindung bringen, dass diese mit dem gesammelten Blut beaufschlagt wird, um einen darin befindlichen Analyten (Glucose) in einer Einmalmessung nachzuweisen. Der Nachweis kann über eine Farbreaktion der streifenförmigen Testeinheiten 20 und eine photometrische Messung in an sich bekannter Weise erfolgen. Hierzu kann zumindest eines der Folienbänder 12, 14 als optisches Fenster aus einem transparenten Material bestehen.

Die Aufnahmezellen 16 sind als im Wesentlichen zweidimensional ausgedehnte Zwischenbereiche zwischen den ebenen Folienbändern 12, 14 freigehalten, um die Testelemente 18, 20 flach aufzunehmen. Die Folienbänder 12, 14 bleiben somit frei von Blistern oder wannenartigen Ausformungen in flächiger Anlage mit den flachen Testelementen, so dass ein kompaktes Aufwickeln oder raumsparendes Falten möglich ist. Beispielsweise können die Stecheinheiten 18 ein Flächenmaß von 5 x 10 mm aufweisen, so dass auch bei einer Anzahl von etwa 100 Stück noch ein akzeptabler Wickeldurchmesser für den Einsatz in einem Handgerät erreicht wird.

Zur Isolierung und gegebenenfalls Fixierung der Testelemente sind die Aufnahmezellen 16 durch linienförmige Verbindungen 28 zwischen den Folienbändern 12, 14 begrenzt. In Fig. 1 ist hierzu eine leiterförmige Struktur aus Schweiß- oder Klebenähten vorgesehen, welche für eine stoffdichte Abdichtung der Aufnahmezellen 16 gegeneinander und gegen Umgebung sorgen.

Durch die gesonderte Anordnung ist es möglich, die verpackten Stecheinheiten 18 unabhängig von den Testeinheiten 20 durch Bestrahlung zu sterilisieren, ohne dass die empfindlichen Testchemikalien der Testeinheiten geschädigt werden. Dies kann über eine nicht gezeigte Abschirmmaske erfolgen, welche eine energiereiche Strahlung (Röntgen- oder Elektronenstrahlen) nur auf die Stecheinheiten 18 durchlässt. Zudem ist es durch die Trennung möglich, die Stecheinheiten 18 für eine effektive Flüssigkeitsaufnahme durch eine Oberflächenbehandlung zu hydrophilisieren, ohne dabei Rücksicht auf die Testchemie nehmen zu müssen. Ein weiterer Vorteil liegt darin, dass die für den Nachweis eingesetzten Chemikalien beim Stechvorgang nicht in den Körper der Untersuchungsperson gelangen können.

Aus den Fig. 2 und 3 ergibt sich eine zweckmäßige Assemblierung einer Bandverpackung 10 mit alternierenden Stech- und Testeinheiten 18, 20. Die vorgefertigten Stecheinheiten 18 werden zwischen den Bändern 12, 14 flach eingeschlossen, wobei die innenseitig an dem Band 12 angeordneten Klebelinien 28 die Aufnahmezellen 16 begrenzen. Außenseitig auf dem Band 14 wird ein durchgehender Teststreifen 30 mittels Klebeband 32 fixiert. Der Teststreifen 30 ist durch Durchbrüche 34 in dem Trägerband 14 in jeder zweiten Aufnahmezelle 16 für die Beaufschlagung mit Körperflüssigkeit bzw. Blut freigestellt, so dass hierdurch die gesonderten Testeinheiten 20 gebildet werden. Mit dem beschriebenen Mehrfolienaufbau wird eine Massenfertigung in hohem Durchsatz von Rolle zu Rolle vereinfacht.

Im Zellenbereich der Testeinheiten 20 können Trockenmittel 36 gegebenenfalls auch als Bandabschnitte enthalten sein. Weiterhin sind in diesen Bereichen Positionierungslöcher 38 in das Band 14 eingestanzt, damit die Stecheinheit 18 nach dem Sammelvorgang genau positioniert werden kann, wie es weiter unten näher erläutert wird. Von besonderem Vorteil ist es, wenn auf einem der Folienbänder 14 insbesondere linien- oder punktförmige Klebestrukturen 40 eine einfache Remagazinierung verbrauchter Stecheinheiten 18 oder integrierter Testelemente erlauben. Möglich ist es auch, dass in den Zellen der Stecheinheiten 18 zu deren lösbarer Halterung zusätzliche Rastpunkte 42 vorgesehen sind.

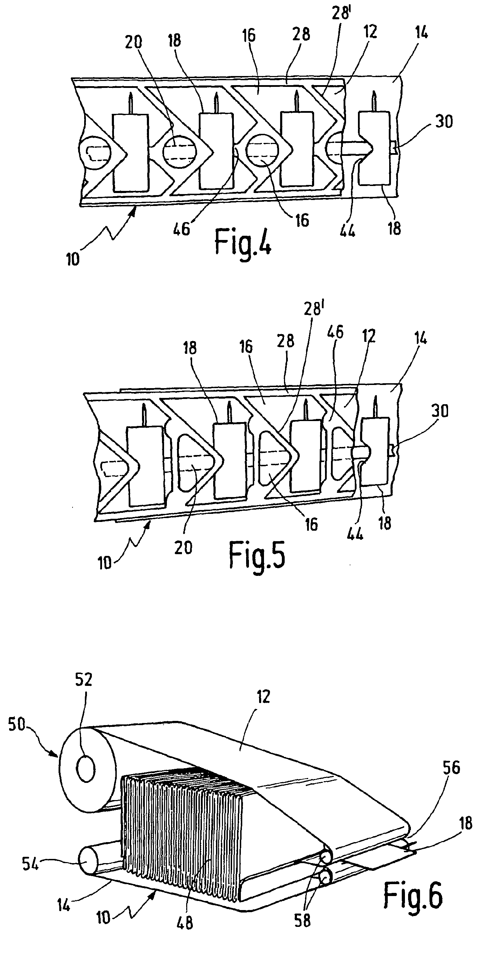

Die Ausführungsformen gemäß Fig. 4 und 5 zeigen ähnliche Folienpackungen 10, wobei gleiche Teile wie vorstehend beschrieben mit gleichen Bezugszeichen versehen sind. Hier zeichnet sich die Verbindungsstruktur 28 zwischen den breitseitig aufeinander liegenden Bändern 12, 14 durch zusätzliche Funktionen aus. Zum einen wird durch einen Eingriff in Ausnehmungen 44 der Stecheinheiten und eine randseitige Anlage an Stützstellen 46 eine lagefeste Fixierung der Stecheinheiten 18 gewährleistet. Zum anderen sorgen schräg zur Bandlängsrichtung verlaufende Verbindungsnähte 28' dafür, ein Aufreißen der Bänder 12, 14 für die Freistellung der Stecheinheiten 18 ohne Kraftspitzen ruckfrei zu ermöglichen.

Fig. 6 veranschaulicht schematisch stark vereinfacht die Bereitstellung einzelner Testelemente bzw. Stecheinheiten 18 aus dem Folienmagazin 10. In der gezeigten Ausführung sind die Folienbänder 12, 14 mit den darin befindlichen Testelementen zick-zack-förmig als Faltpaket 48 bevorratet. Zur sukzessiven Freistellung der Stecheinheiten 18 ist in einem nicht gezeigten Gehäuse eine Bandzugvorrichtung 50 vorgesehen, die zwei Wickelspulen 52, 54 für die Folienbänder 12, 14 sowie diesen vorgeordnete, an einer Spendestelle 56 im seitlichen Abstand voneinander angeordnete Umlenkrollen 58 umfasst. Durch das Auseinanderreißen der Bänder 12, 14 im Bereich der Umlenkrollen 58 werden die Stecheinheiten 18 zur weiteren Verarbeitung freigestellt. Hierbei ist die Rotation der Wickelspulen 52, 54 synchronisiert, um die Bänder 12, 14 immer gespannt zu halten. Eine solche Anordnung kann als Dispenser auch dazu vorgesehen sein, einzelne Einheiten 18 abzuspenden, die dann beispielsweise manuell in ein gesondertes Messgerät eingesetzt werden.

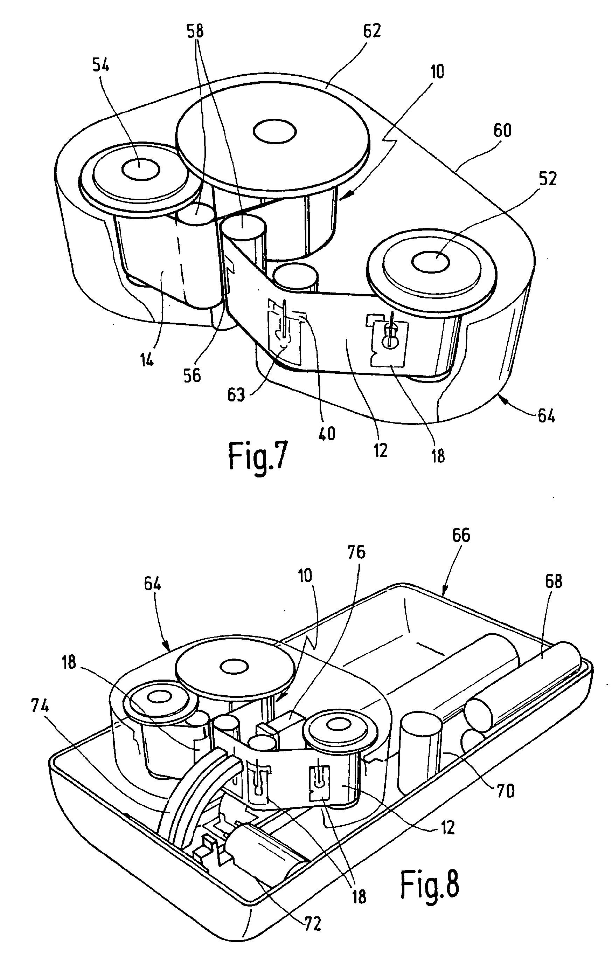

Fig. 7 zeigt eine bevorzugte Anordnung in einem teilweise aufgebrochen dargestellten Magazingehäuse 60. Die Folienverpackung 10 ist dort auf einer Vorratsrolle 62 angeordnet, von der die miteinander verbundenen Bänder 12, 14 über die Umlenkrollen 58 abgezogen und dabei unter Freistellung der Testelemente auseinandergerissen werden. Nach dem Gebrauch werden die Testelemente an einer gegebenenfalls von der abgespendeten Position 56 entfernten Rückgabestelle 63 wieder auf die Innenseite des Bandes 12 zurückverbracht, wobei Klebestreifen 40 die Rückhaltung bewirken. Auf diese Weise können die verbrauchten Testelemente 18 auf der Spule 52 aufgewickelt und damit einfach entsorgt werden.

Die vorstehend beschriebene Testkassette 64 lässt sich als Einwegartikel in ein Handgerät 66 einsetzen, wie es in Fig. 8 vereinfacht gezeigt ist. Ein solches Gerät 66 umfasst alle für die Durchführung einer Glucosemessung notwendigen Betriebsmittel wie Energieversorgung 68, Mess- und Anzeigenelektronik 70 und Aktuatorik 72 für die Handhabung einzelner, aus dem Bandmagazin 10 abgespendeter Stecheinheiten 18 sowie eine Messoptik 76. In der gezeigten Ausführung ist eine Handhabungsvorrichtung 74 als Teil der Aktuatorik 72 vorgesehen, um die Elemente 18 an der Spendestelle 56 aufzunehmen, in eine von den Bändern 12, 14 entfernte quergestellte Stechposition zu bringen, die Stechbewegung über eine Gehäuseöffnung durchzuführen und nach der Blutaufnahme wieder auf das Band 12 zurückzubringen. Dort wird mittels der Messeinheit 76 eine berührungslose optische Messung vorgenommen, um anschließend das ermittelte Ergebnis dem Benutzer anzeigen zu können. Auf diese Weise können in einem vollautomatischen Messablauf auch von Laien Selbstuntersuchungen vorgenommen werden, wobei durch die Magazinierung eine große Anzahl von Tests möglich sind. Die verbrauchten Kassetten 64 lassen sich als komplette Einheit aus dem Gerät 66 entnehmen, ohne dass mit Blut kontaminierte Teile einzeln entsorgt werden müssten.

## Patentansprüche

1. Testmagazin mit zwei sandwichartig miteinander verbundenen, aufwickelbaren Folienbändern (12,14), zwischen denen Aufnahmezellen (16) für Testelemente freigehalten sind, und einer Vielzahl von Testelementen (18,20), welche jeweils eine Stecheinheit (18) zum Einstechen in Körpergewebe und eine Testeinheit (20) zur Beaufschlagung mit Körperflüssigkeit umfassen, **dadurch gekennzeichnet, dass** die Stecheinheiten (18) und Testeinheiten (20) in gesonderten Aufnahmezellen (16) voneinander getrennt angeordnet sind.

2. Testmagazin nach Anspruch 1, **dadurch gekennzeichnet, dass** die Stecheinheiten (18) und Testeinheiten (20) in Bandrichtung alternierend oder quer dazu paarweise benachbart in zugeordneten Aufnahmezellen (16) angeordnet sind.

3. Testmagazin nach Anspruch 1 oder 2, **dadurch gekennzeichnet, dass** die Stecheinheiten (18) in den zugeordneten Aufnahmezellen (16) durch Bestrahlung vorzugsweise über eine Maske zur Abschirmung der Testeinheiten (20) sterilisiert sind.

4. Testmagazin nach einem der Ansprüche 1 bis 3, **dadurch gekennzeichnet, dass** die Folienbänder (12,14) über ihre Länge mit einem Testband (30) verbunden sind, und dass das Testband (30) durch Durchbrüche (34) der Folienbänder (12,14) unter Bildung der Testeinheiten (20) zellenweise freigestellt ist.

5. Testmagazin nach einem der Ansprüche 1 bis 4, **dadurch gekennzeichnet, dass** in den Aufnahmezellen (16) für die Testeinheiten (20) ein Trockenmittel (36) eingelagert ist.

6. Testmagazin nach einem der Ansprüche 1 bis 5, **dadurch gekennzeichnet, dass** zumindest die Stecheinheiten (18) durch Transportmittel (50,74) aus ihrer jeweiligen Aufnahmezelle entnehmbar und in eine von den Folienbändern (12,14) getrennte Arbeitsposition bewegbar sind.

7. Testmagazin nach Anspruch 6, **dadurch gekennzeichnet, dass** die Transportmittel (50,74) eine Bandzugvorrichtung (50) zum Auseinanderziehen der Folienbänder (12,14) in verschiedene Richtungen umfassen.

8. Testmagazin nach Anspruch 7, **dadurch gekennzeichnet, dass** die Bandzugvorrichtung (50) zwei an einer Spendestelle (56) im seitlichen Abstand voneinander angeordnete, in entgegengesetzte Richtungen drehbare oder feststehende Umlenkzylinder (58) und den Umlenkzylindern (58) nachgeordnete Aufwickelspulen (52,54) für die Folienbänder (12,14) aufweist.

9. Testmagazin nach einem der Ansprüche 6 bis 8, **dadurch gekennzeichnet, dass** die Transportmittel (50,74) zum Rückführen einer Stecheinheit (18) in eine Wirkverbindung mit einer auf den Folienbändern (12,14) befindlichen Testeinheit (20) und/oder Entsorgungsstelle ausgebildet sind.

10. Testmagazin nach einem der Ansprüche 6 bis 9, **dadurch gekennzeichnet, dass** die Transportmittel (50,74) eine Handhabungsvorrichtung (74) zum Aufgreifen und Positionieren einer durch Auseinanderziehen der Folienbänder (12,14) an einer Spendestelle (56) freigestellten Stecheinheit (18) umfassen.

11. Testmagazin nach Anspruch 10, **dadurch gekennzeichnet, dass** zumindest eines der Folienbänder (12,14) Positionierlöcher (38) insbesondere im Bereich der Aufnahmezellen (16) der Testeinheiten (20) für den Eingriff der Handhabungsvorrichtung (74) aufweist.

12. Testmagazin nach einem der Ansprüche 6 bis 11, **dadurch gekennzeichnet, dass** die Stecheinheiten (18) mit den Testeinheiten (20) als integrierte Testelemente (18,20) körperlich verbunden sind.

13. Testmagazin nach einem der vorhergehenden Ansprüche, **dadurch gekennzeichnet, dass** zumindest eines der Folienbänder (12,14) mit einer Rücknahmestruktur (40) zur vorzugsweise klebenden oder klemmenden Fixierung verbrauchter Stechelemente (18) versehen ist.

14. Testmagazin nach einem der Ansprüche 1 bis 13, **dadurch gekennzeichnet, dass** die Stecheinheiten (18) als Flachmaterialteile flach zwischen den Folienbändern (12,14) angeordnet sind.

15. Testmagazin nach einem der Ansprüche 1 bis 14, **dadurch gekennzeichnet, dass** die Folienbänder (12,14) eben ausgebildet sind und frei von Blistern flächig gegen die Stecheinheiten (18) anliegen.

16. Testmagazin nach einem der Ansprüche 1 bis 15, **dadurch gekennzeichnet, dass** die an einer Spendestelle (56) freigestellten Stecheinheiten (18) an einer davon entfernten Rückgabestelle auf eines der Folienbänder (12,14) zurückdispensiert werden.

17. Testmagazin nach einem der Ansprüche 1 bis 16, **dadurch gekennzeichnet, dass** die Stecheinheiten (18) eine vorzugsweise durch einen halboffenen Kanal gebildete Kapillarstruktur (24) zum Sammeln von Körperflüssigkeit aufweisen.

18. Testmagazin nach einem der Ansprüche 1 bis 17, **dadurch gekennzeichnet, dass** die Stecheinheiten (18) zur Weitergabe von aufgenommener Körperflüssigkeit auf die Testeinheiten (20) ausgebildet sind.

19. Testmagazin nach einem der Ansprüche 1 bis 18, **dadurch gekennzeichnet, dass** die Aufnahmezellen (16) durch linienförmige Folienverbindungen (28), vorzugsweise Schweiß- oder Klebenähte zwischen den Folienbändern (12,14) begrenzt sind.

20. Testmagazin nach Anspruch 19, **dadurch gekennzeichnet, dass** die Aufnahmezellen (16) durch die Folienverbindungen (28) gegeneinander und gegen die Umgebung stoffdicht abgedichtet sind.

21. Testmagazin nach Anspruch 19 oder 20, **dadurch gekennzeichnet, dass** die linienförmigen Folienverbindungen (28') schräg zur Bandlängsrichtung der Folienbänder (12,14) verlaufen.

22. Testmagazin nach einem der Ansprüche 1 bis 21, **dadurch gekennzeichnet, dass** die Stecheinheiten (18) durch formschlüssig umrandende oder in Ausnehmungen (44) eingreifende Folienverbindungen (28) lagefest in ihrer zugeordneten Aufnahmezelle (16) fixiert sind.

23. Testmagazin nach einem der Ansprüche 1 bis 22, **dadurch gekennzeichnet, dass** mindestens eines der Folienbänder (12,14) ein transparentes Messfenster für eine optische Vermessung der Testeinheiten (20) aufweist oder bildet.

24. Testmagazin nach einem der Ansprüche 1 bis 23, **dadurch gekennzeichnet, dass** die Folienbänder (12,14) mit den darin befindlichen Testelementen (18,20) in einer Zickzack-Faltung als Faltpaket (48) bereitgehalten sind.

25. Testmagazin nach einem der Ansprüche 1 bis 24, **gekennzeichnet durch** eine die Folienbänder (12,14) mit den darin befindlichen Testelementen (18,20) aufnehmende Kassette (64).

26. Testmagazin nach Anspruch 25, **dadurch gekennzeichnet, dass** die Kassette (64) als Spender für Testelemente (18,20) ausgebildet ist, so dass die einzeln abgespendeten Testelemente in ein gesondertes Testgerät einsetzbar sind.

27. Testmagazin nach einem der Ansprüche 1 bis 26, **dadurch gekennzeichnet, dass** die Testeinheiten (20) zum Nachweis eines Analyten in der Körperflüssigkeit ausgebildet sind.

28. Verfahren zur Verarbeitung eines Testmagazins (10) nach einem der Ansprüche 1 bis 25, bei welchem die Stecheinheiten (18) gegebenenfalls in Verbindung integrierten Testeinheiten (20) durch Auseinanderziehen der Folienbänder (12,14) einzeln freigestellt, sodann in eine von der zugehörigen Aufnahmezelle (16) entfernte Arbeitsposition bewegt und anschließend wieder auf einem der Folienbänder (12,14) remagaziniert werden.

## Claims

1. Test magazine comprising two spoolable foil tapes (12, 14) joined together in a sandwich-like manner and between which holding cells (16) are kept free for test elements, and a plurality of test elements (18, 20) which each comprise a lancing unit (18) for puncturing body tissue and a test unit (20) to which body fluid can be applied, **characterized in that** the lancing units (18) and test units (20) are arranged separately from one another in separate holding cells (16).

2. Test magazine according to claim 1, **characterized in that** the lancing units (18) and test units (20) are arranged alternately in the tape direction or adjacent to one another in pairs across the tape direction in associated holding cells (16).

3. Test magazine according to claim 1 or 2, **characterized in that** the lancing units (18) in the associated holding cells (16) are sterilized by irradiation preferably through a mask in order to screen the test units (20).

4. Test magazine according to one of the claims 1 to 3, **characterized in that** the foil tapes (12, 14) are joined to a test tape (30) over their length and that the test tape (30) is exposedcell by cell through perforations (34) in the foil tapes (12, 14) to form the test units (20).

5. Test magazine according to one of the claims 1 to 4, **characterized in that** a desiccant (36) is stored in the holding cells (16) for the test units (20).

6. Test magazine according to one of the claims 1 to 5, **characterized in that** at least the lancing units (18) can be removed by transport means (50, 74) from their respective holding cell and can be moved into a working position which is separate from the foil tapes (12, 14).

7. Test magazine according to claim 6, **characterized in that** the transport means (50, 74) comprises a tape tensioning device (50) to pull apart the foil tapes (12, 14) in different directions.

8. Test magazine according to claim 7, **characterized in that** the tape tensioning device (50) has two deflecting cylinders (58) that can rotate in opposite directions or are stationary and are arranged laterally spaced apart from one another at a dispensing site (56) and has take-up spools (52, 54) for the foil tapes (12, 14) downstream of the deflecting cylinders (58).

9. Test magazine according to one of the claims 6 to 8, **characterized in that** the transport means (50, 74) are in operative connection with a test unit (20) and/or disposal site located on the foil tapes (12, 14) in order to return a lancing unit (18).

10. Test magazine according to one of the claims 6 to 9, **characterized in that** the transport means (50, 74) comprise a handling device (74) to pick up and position a lancing unit (18) that has been released by pulling apart the foil tapes (12, 14) at a dispensing position (56).

11. Test magazine according to claim 10, **characterized in that** at least one of the foil tapes (12, 14) has positioning holes (38) especially in the region of the holding cells (16) for the test units (20) to enable the handling device (74) to engage.

12. Test magazine according to one of the claims 6 to 11, **characterized in that** the lancing units (18) are physically joined to the test units (20) as integrated test elements (18, 20).

13. Test magazine according to one of the previous claims, **characterized in that** at least one of the foil tapes (12, 14) is provided with a withdrawal structure (40) for the preferably adhesive or clamping fixation of used lancing elements (18).

14. Test magazine according to one of the claims 1 to 13, **characterized in that** the lancing units (18) are arranged flat between the foil tapes (12, 14) as flat material components.

15. Test magazine according to one of the claims 1 to 14, **characterized in that** the foil tapes (12, 14) have a planar design and rest laminarly against the lancing units (18) free of blisters.

16. Test magazine according to one of the claims 1 to 15, **characterized in that** the lancing units (18) released at a dispensing site (56) can be redispensed at a return position on one of the foil tapes (12, 14).

17. Test magazine according to one of the claims 1 to 16, **characterized in that** the lancing units (18) have a capillary structure (24) that is preferably formed by a semi-open channel to collect body fluid.

18. Test magazine according to one of the claims 1 to 17, **characterized in that** the lancing units (18) are formed in order to transfer collected body fluid to the test units (20).

19. Test magazine according to one of the claims 1 to 18, **characterized in that** the holding cells (16) are delimited by linear foil connections (28), preferably welded or adhesive seams between the foil tapes (12, 14).

20. Test magazine according to claim 19, **characterized in that** the holding cells (16) are sealed against one another and against the environment in a material-tight manner by the foil connections (28).

21. Test magazine according to claim 19 or 20, **characterized in that** the linear foil connections (28') extend obliquely to the tape longitudinal direction of the foil tapes (12, 14).

22. Test magazine according to one of the claims 1 to 21, **characterized in that** the lancing units (18) are immobilized in a fixed position in their assigned holding cell (16) by complementarily engaging bordering foil connections or foil connections (28) that engage in recesses (44).

23. Test magazine according to one of the claims 1 to 22, **characterized in that** at least one of the foil tapes (12, 14) has or forms a transparent measuring window for an optical measurement of the test units (20).

24. Test magazine according to one of the claims 1 to 23, **characterized in that** the foil tapes (12, 14) with the test elements (18, 20) located therein are held ready as a folded package (48) in a zigzag folding.

25. Test magazine according to one of the claims 1 to 24, **characterized by** a cassette (64) that receives the foil tapes (12, 14) with the test elements (18, 20) located therein.

26. Test magazine according to claim 25, **characterized in that** the cassette (64) is designed as a dispenser for test elements (18, 20) such that the individually dispensed test elements can be used in a separate test device.

27. Test magazine according to one of the claims 1 to 26, **characterized in that** the test units (20) are designed to detect an analyte in the body fluid.

28. Process for processing a test magazine (10) according to one of the claims 1 to 25 in which the lancing units (18) optionally in conjunction with integrated test units (20) are individually released by pulling apart the foil tapes (12, 14), are then moved into a working position that is distant from the associated holding cell (16) and are subsequently stored again on one of the foil tapes (12, 14).

## Revendications

1. Magasin de test comprenant deux bandes en feuille (13, 14) enroulables, reliées l'une à l'autre en sandwich, entre lesquelles sont réservées des cellules de réception (16) pour des éléments de test, et une pluralité d'éléments de test (18, 20) qui comprennent respectivement une unité de piqûre (18) pour pratiquer une piqûre dans le tissu corporel et une unité de test (20) pour la solliciter avec un fluide organique, **caractérisé en ce que** les unités de piqûre (18) et les unités de test (20) sont disposées à l'écart l'une de l'autre dans des cellules de réception séparées (16).

2. Magasin de test selon la revendication 1, **caractérisé en ce que** les unités de piqûre (18) et les unités de test (20) sont disposées en position adjacente par paires en alternance dans la direction de la bande ou transversalement par rapport à cette direction, dans des cellules de réception correspondantes (16).

3. Magasin de test selon la revendication 1 ou 2, **caractérisé en ce que** les unités de piqûre (18) sont stérilisées dans les cellules de réception correspondantes (16) par exposition à un rayonnement, de préférence par l'intermédiaire d'un masque pour la protection des unités de test (20).

4. Magasin de test selon l'une quelconque des revendications 1 à 3, **caractérisé en ce que** les bandes en film (12, 14) sont reliées, sur leur longueur à une bande de test (30), et **en ce que** la bande de test (30) est laissée libre en forme de cellules via des perforations (34) des bandes en film (12, 14) en formant des unités de test (20).

5. Magasin de test selon l'une quelconque des revendications 1 à 4, **caractérisé en ce qu'**un dessiccatif (36) est incorporé dans les cellules de réception (16) pour les unités de tests (20).

6. Magasin de test selon l'une quelconque des revendications 1 à 5, **caractérisé en ce qu'**au moins les unités de piqûre (18) peuvent être retirées via des moyens de transport (50, 74) de leurs cellules de réception respectives et peuvent être amenées dans une position de travail à l'état séparé des bandes en film (12, 14).

7. Magasin de test selon la revendication 6, **caractérisé en ce que** les moyens de transport (50, 74) comprennent un dispositif de traction de bande (50) pour séparer les bandes en film (12, 14) dans des directions différentes.

8. Magasin de test selon la revendication 7, **caractérisé en ce que** le dispositif de traction de bande (50) présente deux cylindres de renvoi (58) rotatifs dans des directions opposées ou fixes, disposés, à un endroit de distribution (58), en étant écartés latéralement l'un de l'autre, et des bobines d'enroulement (52, 54) pour les bandes en film (12, 14), montées à la suite des cylindres de renvoi.

9. Magasin de test selon l'une quelconque des revendications 6 à 8, **caractérisé en ce que** les moyens de transports (50, 74) sont réalisés pour le renvoi d'une unité de piqûre (18) dans une liaison de travail avec une unité de test (20) se trouvant sur les bandes en film (12, 14) et/ou dans un endroit d'élimination.

10. Magasin de test selon l'une quelconque des revendications 6 à 9, **caractérisé en ce que** les moyens de transports (50, 74) comprennent un dispositif de manipulation (74) pour saisir et positionner une unité de piqûre (18) libérée à un endroit de distribution (56) via la séparation des bandes en film (12, 14).

11. Magasin de test selon la revendication 10, **caractérisé en ce qu'**au moins une des bandes en film (12, 14) présente des trous de positionnement (38) en particulier dans la zone des cellules de réception (16) des unités de test (20) pour la préhension du dispositif de manipulation (74).

12. Magasin de test selon l'une quelconque des revendications 6 à 11, **caractérisé en ce que** les unités de piqûre (18) sont reliées intégralement aux unités de test (20) sous la forme d'éléments de test intégrées (18, 20).

13. Magasin de test selon l'une quelconque des revendications précédentes, **caractérisé en ce qu'**au moins une des bandes en film (12, 14) est munie d'une structure de reprise (40) pour la fixation de préférence par collage ou par serrage d'éléments de piqûre usagés (18).

14. Magasin de test selon l'une quelconque des revendications 1 à 13, **caractérisé en ce que** les unités de piqûre (18) sont disposées à plat sous la forme de parties de matières plates entre les bandes en film (12, 14).

15. Magasin de test selon l'une quelconque des revendications 1 à 14 **caractérisé en ce que** les bandes en film (12, 14) sont réalisées pour être planes et viennent se disposer en l'absence de cloques à fleur contre les unités de piqûre (18).

16. Magasin de test selon l'une quelconque des revendications 1 à 15, **caractérisé en ce que** les unités de piqûre (18) libérées à un endroit de distribution (56) sont distribuées en retour à un endroit de restitution qui en est écarté sur une des bandes en film (12, 14).

17. Magasin de test selon l'une quelconque des revendications 1 à 16, **caractérisé en ce que** les unités de piqûre (18) présentent une structure capillaire (24) formée de préférence par un canal à demi ouvert pour la récupération du liquide organique.

18. Magasin de test selon l'une quelconque des revendications 1 à 17, **caractérisé en ce que** les unités de piqûre (18) sont réalisées pour la transmission aux unités de test (20) du liquide organique récupéré.

19. Magasin de test selon l'une quelconque des revendications 1 à 18, **caractérisé en ce que** les cellules de réception (16) sont délimitées par des raccords de feuilles de configuration linéaire (28), de préférence des cordons de soudure ou de collage entre les bandes en film (12, 14).

20. Magasin de test selon la revendication 19, **caractérisé en ce que** des cellules de réception (16) sont rendues étanches via les raccords de feuilles (28) les unes par rapport aux autres et par rapport à l'environnement.

21. Magasin de test selon la revendication 19 ou 20, **caractérisé en ce que** les raccords de feuilles de configuration linéaire (28') s'étendent en inclinaison par rapport à la direction longitudinale des bandes en film (12, 14).

22. Magasin de test selon l'une quelconque des revendications 1 à 21, **caractérisé en ce que** les unités de piqûre (18) sont fixées à demeure dans leur cellule de réception correspondante (16) via des raccords en film (28) périphériques épousant la forme ou engrenés dans des évidements (44).

23. Magasin de test selon l'une quelconque des revendications 1 à 22, **caractérisé en ce qu'**au moins une des bandes en film (12, 14) présente ou forme une fenêtre de mesure transparente pour une mesure optique des unités de test (20).

24. Magasin de test selon l'une quelconque des revendications 1 à 23 **caractérisé en ce que** les bandes en film (12, 14) dans lesquelles sont disposés les éléments de test (18, 20) sont disponibles en étant pliés en zigzag sous la forme d'un paquet plié (48).

25. Magasin de test selon l'une quelconque des revendications 1 à 24 **caractérisé par** une cassette (64) dans laquelle viennent se loger les bandes en film (12, 14) dans lesquelles se trouvent les éléments de test (18,20).

26. Magasin de test selon la revendication 25, **caractérisé en ce que** la cassette (64) est réalisée sous la forme de distributeur pour des éléments de test (18, 20), de telle sorte que les éléments de test distribués de manière individuelle peuvent venir s'insérer dans un appareil de test séparé.

27. Magasin de test selon l'une quelconque des revendications 1 à 26 **caractérisé en ce que** les unités de test (20) sont réalisées pour le décèlement d'un analyte dans le fluide organique.

28. Procédé pour le traitement d'un magasin de test selon l'une quelconque des revendications 1 à 25, dans lequel les unités de piqûre (18) sont libérées de manière individuelle le cas échéant en liaison avec des unités de test intégrées (20) en exerçant une traction sur les bandes en film (12, 14) sont ainsi amenées dans une position de travail à l'écart de la cellule de réception correspondante (16) et sont ensuite réemmagasinées sur une des bandes en film (12, 14).
